# EUROPEAN PATENT SPECIFICATION

(11) **EP 2 293 751 B1**
(45) Date of publication and mention of the grant of the patent: **10.01.2018**
(21) Application number: 09798502.2
(22) Date of filing: 23.06.2009
(51) Int. Cl.: A61F 13/00

(54) **MULTIDIRECTIONAL MUCOSAL DELIVERY DEVICES AND METHODS OF USE**
VORRICHTUNGEN ZUR MULTIDIREKTIONALEN SCHLEIMHAUTVERABREICHUNG UND ANWENDUNGSVERFAHREN DAFÜR
DISPOSITIFS D'ADMINISTRATION MUCOSALE À DIRECTIONS MULTIPLES ET LEURS PROCÉDÉS D'UTILISATION

(30) Priority: 23.06.2008 US 74918
(43) Date of publication of application: 16.03.2011
(73) Proprietor: BioDelivery Sciences International, Inc., Raleigh, NC 27612 (US)
(72) Inventor: VASISHT, Niraj, Cary, NC 27518 (US); FINN, Andrew, Raleigh, NC 27601 (US)
(74) Representative: Hoffmann Eitle
(86) International application number: PCT/US2009/048325
(87) International publication number: WO 2010/008863

(56) References cited:
- EP-A2- 0 250 187
- WO-A1-01/91553
- WO-A1-2008/025791
- WO-A1-2008/068471
- WO-A2-2005/039499
- WO-A2-2008/011194
- US-A1- 2001 046 511
- US-A1- 2005 196 440
- US-A1- 2006 039 957
- US-A1- 2006 198 881
- US-A1- 2006 241 017
- US-A1- 2007 148 097
- US-B2- 6 344 212

## Description

### RELATED APPLICATION

This application claims the benefit of and priority to U.S. Provisional Application No. 61/074,918, filed on June 23, 2008.

### SUMMARY OF THE INVENTION

Disclosed herein are pharmaceutical delivery devices for use in methods of transmucosally delivering buprenorphine to a subject and methods of making such devices. Without wishing to be bound by any particular theory, it is believed that the pharmaceutical delivery devices of the present invention allow improved loading of buprenorphine, and in some embodiments, enhanced uptake of buprenorphine in a subject by simultaneously enabling at least two sites of adhesion and drug delivery for each device. For example, exemplary pharmaceutical delivery devices of the present invention may include at least two mucoadhesive surfaces such that buprenorphine is absorbed across both mucosal surfaces simultaneously.

Provided herein are devices for use in methods of transmucosally delivering buprenorphine to a subject in need thereof, by administering to said subject in need thereof a thin and flexible pharmaceutical delivery device. The device is composed of a first mucoadhesive surface for transmucosal delivery of buprenorphine and a second mucoadhesive surface, opposing the first mucoadhesive surface, for transmucosal delivery of buprenorphine. The device also includes an abusable drug and an antagonist incorporated therein, wherein the abusable drug is buprenorphine and the antagonist is naloxone. The first and second mucoadhesive surfaces are defined by at least one thin and flexible mucoadhesive film layer. An effective amount of buprenorphine is delivered to a subject upon administration. Both mucoadhesive surfaces are formulated for the transmucosal delivery of buprenorphine. In certain other embodiments, the device is bioerodable. The device, for instance, can erode in the oral cavity.

In some embodiments, the device includes a first and a second mucoadhesive surface that are on opposing sides of a single mucoadhesive layer. That is, the first and second mucoadhesive surfaces are defined by a single mucoadhesive layer. In other embodiments, the device is a multi-layered device comprising at least two mucoadhesive layers, wherein the first and second mucoadhesive surfaces are defined by at least a first and a second thin and flexible mucoadhesive film layer. There are certain embodiments wherein the first and second mucoadhesive surfaces are on opposing sides of the two outermost layers.

In some embodiments, the device contains at least one intermediate layer. For example a device having two thin and flexible mucoadhesive film layers may have an intermediate layer disposed between the two mucoadhesive film layers, such that the two mucoadhesive surfaces of the mucoadhesive film layers are opposing each other.

In some embodiments, the device further contains an abuse-resistant matrix and an antagonist associated with the abuse-resistant matrix such that abuse of buprenorphine is prevented. In some embodiments, the intermediate layer includes the abuse-resistant matrix and an antagonist associated with the abuse-resistant matrix such that the antagonist is substantially transmucosally unavailable. In some embodiments, the abuse-resistant matrix is disposed between a first mucoadhesive layer and a second mucoadhesive layer. In some embodiments, antagonist of the abuse-resistant matrix is encapsulated within the device. In certain embodiments the antagonist is encapsulated within at least one mucoadhesive layer. Some embodiments provide for the microencapsulation of the antagonist in the device such as in the intermediate layer or in any one of the mucoadhesive layers.

In some embodiments, the device also includes an intermediate layer disposed between the two mucoadhesive layers or surfaces wherein the intermediate layer is occlusive to buprenorphine. The intermediate layer, in some embodiments, is occlusive such that buprenorphine incorporated into the first and/or second mucoadhesive layer does not diffuse from that layer into the other layer. An occlusive layer prohibits diffusion of buprenorphine from the first mucoadhesive layer to the second mucoadhesive layer or from the second mucoadhesive layer to first mucoadhesive layer. In some embodiments, the abuse-resistant matrix is incorporated into the intermediate layer. In other embodiments, the abuse-resistant matrix is incorporated into the intermediate layer by encapsulation.

Other embodiments are also provided wherein buprenorphine is incorporated into the first mucoadhesive layer or surface, the second mucoadhesive layer or surface, or any combination of the layers or surfaces. The active agent is an abusable drug, i.e. buprenorphine.

In other embodiments, the device for use in the above methods include an abuse-resistant-matrix and an antagonist associated with the abuse-resistant matrix such that abuse of buprenorphine is prevented. The antagonist can include naloxone. In some embodiments, the subject experiences a state of moderate withdrawal.

The devices also encompass embodiments wherein buprenorphine is transmucosally delivered to two or more mucosal surfaces. Other embodiments provide for simultaneous or sequential transmission to the mucosal surfaces.

Some embodiments provide for the administration of the device to a subject by applying the device to a mucosal cavity of the subject such that there is adhesion of the delivery device to at least two surfaces of the mucosal cavity and diffusion of buprenorphine across at least two surfaces of the mucosal cavity.

In some embodiments, an effective amount of buprenorphine is delivered to the subject in less than about 1 hour. In other embodiments, the delivery time is less than about 45 minutes, or less than about 30 minutes, or less than about 20 minutes, or less than about 15 minutes. In some embodiments, the effective residence time is about 20 minutes or about 30 minutes.

The methods include certain embodiments such that upon administration of the device the subject does not experience significant nausea.

In some embodiments, onset of pain relief is achieved in less than about 1.0 hour, or 0.5 hours, 0.25 hours, or 0.1 hours.

The active agent in the device is buprenorphine and ranges in quantity from about 0.1 mg to about 60 mg. Tₘₐₓ is less than about 100 minutes in some embodiments, while in other, Tₘₐₓ is less than about 80 minutes, less than about 60 minutes, less than about 30 minutes or less than about 20 minutes. Some embodiments provide for about 30%, 40%, 50%, 60%, or 70% bioavailability of the buprenorphine.

In certain embodiments, the device for use in the above methods contain about 16 mg of buprenorphine and provide a Cₘₐₓ of about 5.95ng/mL or 8.0 ng/mL. In some other embodiments Cₘₐₓ is about 3.0 ng/mL or about 4.5 ng/mL when the device contains 8 mg of buprenorphine. When the device contains about 4 mg of buprenorphine, certain embodiments provide a Cₘₐₓ of about 1.84 ng/mL or about 2.5 ng/mL.

Certain devices for use in other methods are also provided herein for the treatment of addiction to certain active agents, e.g., an opioid, by the administration of any of the devices disclosed herein.

In certain other embodiments, devices for use in methods for the treatment of pain are provided. In some embodiments the pain is breakthrough cancer pain.

In certain embodiments, the above devices deliver an effective amount of buprenorphine to the subject in a one unit dose.

### BRIEF DESCRIPTION OF THE DRAWINGS

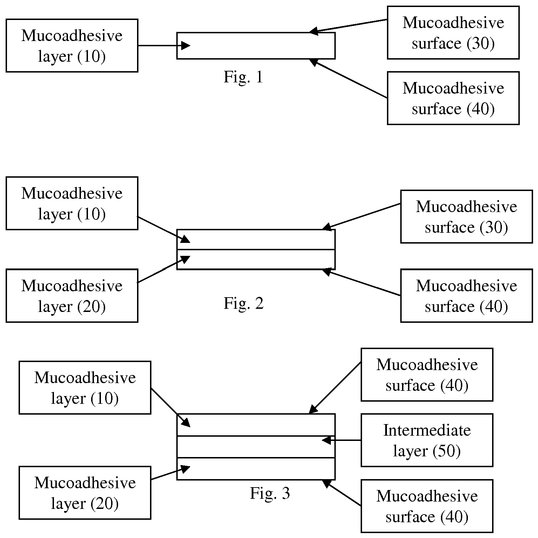

**Figure 1** is a depiction of an exemplary double-sided mucoadhesive pharmaceutical delivery device, having a single thin and flexible mucoadhesive film layer (10), wherein both top (30) and bottom (40) surfaces are capable of mucosal adhesion.
**Figure 2** is a depiction of an exemplary bi-layered double sided mucoadhesive device, having two mucoadhesive layers (10 and 20), wherein both top (30) and bottom (40) surfaces are capable of mucosal adhesion.
**Figure 3** is a depiction of an exemplary tri-layered double-sided mucoadhesive device having two outermost mucoadhesive layers (10 and 20), wherein the two outermost layers have opposing mucoadhesive surfaces (30 and 40). The top and bottom surfaces are capable of mucosal adhesion. The intermediate layer (50) can include naloxone.

### DETAILED DESCRIPTION OF THE INVENTION

In order to more clearly and concisely describe the subject matter of the claims, the following definitions are intended to provide guidance as to the meaning of terms used herein.

As used herein, the articles "a" and "an" mean "one or more" or "at least one," unless otherwise indicated. That is, reference to any element of the present invention by the indefinite article "a" or "an" does not exclude the possibility that more than one of the element is present.

The terms "abusable drug" or "drug" as used interchangeably herein, refers to any pharmaceutically active substance or agent that has the ability to promote abuse, high tolerance with extended use, and/or chemical or physical dependency. Abusable drugs include, but are not limited to, drugs for the treatment of pain such as an opioid analgesic, *e.g.*, and opioid or an opiate.

As used herein, the term "antagonist" refers to a moiety that renders the active agent unavailable to produce a pharmacological effect, inhibits the function of an agonist, *e*.*g*., an abusable drug, at a specific receptor, or produces an adverse pharmacological effect. Without wishing to be bound by any particular theory, it is believed that antagonists generally do not alter the chemical structure of the abusable drug itself, but rather work, at least in part, by an effect on the subject, e.g., by binding to receptors and hindering the effect of the agonist. Antagonists can compete with an agonist for a specific binding site (competitive antagonists) and/or can bind to a different binding site from the agonist, hindering the effect of the agonist via the other binding site (non- competitive antagonists). Non-limiting examples of antagonists include opioid neutralizing antibodies; narcotic antagonists such as naloxone, naltrexone and nalmefene; dysphoric or irritating agents such as scopolamine, ketamine, atropine or mustard oils; or any combinations thereof. In one embodiment, the antagonist is naloxone or naltrexone.

The term "abuse-resistant matrix" refers generally to a matrix with which an antagonist to an abusable drug is associated. An abuse resistant matrix is a matrix that effectively releases the antagonist when the device is used in an abusive manner (e.g., dissolved in water in an attempt to extract the drug, solubilized, opened, chewed and/or cut apart) so that, *e.g*., the antagonist is co-extracted and alters or blocks the effect the drug. However, when used as intended, e.g., in a non-abusive manner, the abuse-resistant matrix does not effectively release the antagonist. *E*.*g*., the antagonist instead is retained within the matrix and is delivered to the gastrointestinal tract where it is not readily absorbed such that any amount of antagonist delivered systemically through the mucosa and/or the GI tract does not significantly block or alter the effect of the drug.

As used herein, the term "abusive manner" refers to the use of the delivery device in a manner not intended, *e.g.,* in a non-transmucosal manner or in a manner not otherwise prescribed by a physician. In some embodiments, the abusive manner includes extraction of the drug from the delivery device for oral or parenteral administration. As used herein, "non-abusive manner" refers to the use of the delivery device for its intended purpose, *e*.*g*., transmucosal administration of the drug. In some cases, a portion of the drug will unintentionally be delivered non-transmucosally, e.g., orally through the dissolution of a portion of the device. Such inadvertent or unintentional delivery is not indicative of use in an abusive manner.

As used herein, "treatment" of a subject includes the administration of a drug to a subject with the purpose of preventing, curing, healing, alleviating, relieving, altering, remedying, ameliorating, improving, stabilizing or affecting a disease or disorder, or a symptom of a disease or disorder (*e.g.*, to alleviate pain).

The term "subject" refers to living organisms such as humans, dogs, cats, and other mammals. Administration of the buprenorphine included in the devices provided herein can be carried out at dosages and for periods of time effective for treatment of a subject.

An "effective amount" of buprenorphine necessary to achieve a therapeutic effect may vary according to factors such as the age, sex, and weight of the subject. Dosage regimens can be adjusted to provide the optimum therapeutic response. For example, several divided doses may be administered daily or the dose may be proportionally reduced as indicated by the exigencies of the therapeutic situation. Similarly, effective amounts of antagonist to an abusable drug will vary according to additional factors such as the amount of abusable drug included in the devices.

As used herein, the term "incorporated" as used with respect to incorporation of buprenorphine into the devices herein disclosed, or any layer of the devices, refers to buprenorphine being disposed within, associated with, mixed with, or otherwise part of a transmucosal device, e.g., within one or more layers of a multilayered device or existing as a layer or coating of the device. It is to be understood that the mixture, association or combination need not be regular or homogeneous.

In the present invention, pharmaceutical delivery devices are provided for transmucosally delivering buprenorphine to a subject in need thereof. The device includes a thin and flexible film that possesses mucoadhesive properties making it suitable for adhesion to mucosal surfaces. For example, the film's properties permit administration of buprenorphine to a subject by adhering the device to two or more mucosal surfaces of the buccal cavity of a subject. The device includes a thin flexible film, having a first mucoadhesive surface for the directional diffusion of buprenorphine across a first mucosal surface; a second mucoadhesive surface, opposing the first mucoadhesive surface, for the directional diffusion of buprenorphine across a second mucosal surface; and at least an abusable drug incorporated into the device such that there are multi-directional diffusions across at least two mucosal surfaces. The thin and flexible mucoadhesive film, interchangeably described herein as a mucoadhesive layer, includes at least two mucoadhesive surfaces. The first mucoadhesive surface is situated opposite the second mucoadhesive surface and allows directional diffusion of buprenorphine across a first mucosal surface to which it is adhered. The second mucoadhesive surface, adhered to a second mucosal surface, allows directional diffusion of buprenorphine across the second mucosal surface.

While in some embodiments, the device includes a single mucoadhesive layer, in others the device is a multi-layered device such as a device having two mucoadhesive layers. The two mucoadhesive layers are disposed such that the device retains two opposing mucoadhesive surfaces. For example, the two layers of a two-layered device may be coated, one on top of the other, such that the two outermost surfaces are mucoadhesive. It is also contemplated that any other method known to one skilled in the art for the preparation of a multi-layered device may be employed. For example, certain lamination processes may be employed in the preparation of the multi-layered device.

In other embodiments, the device includes one or more intermediate layers disposed between the two outermost mucoadhesive surfaces. The intermediate layer is formulated such that in certain embodiments it provides stability to the device. In other embodiments, the intermediate layer is formed such that it is an occlusive barrier preventing the diffusion of buprenorphine from one layer of the device to another. For example, where the device has two mucoadhesive layers, an intermediate layer can be placed between the two mucoadhesive layers to prevent the diffusion of buprenorphine incorporated into the first and/or second mucoadhesive layer, from diffusing into the other layer. An occlusive intermediate layer prevents diffusion of buprenorphine from a first mucoadhesive layer of the device to a second mucoadhesive layer of the device. The intermediate layer may also be used to incorporate an abuse-resistant matrix or additional active agents. Multi-layered devices that have more than two mucoadhesive layers are also contemplated. The device, for example, may contain between 2 and 10 layers. All layers situated between the two outermost mucoadhesive layers are herein considered intermediate layers. Accordingly, an intermediate layer may also be a mucoadhesive layer, possessing all the same, or different, properties of the outermost mucoadhesive layers.

In some embodiments, the device includes two mucoadhesive layers and two active agents wherein the first active agent is incorporated into the first mucoadhesive layer and the second active agent is incorporated into the second mucoadhesive layer. In some embodiments, the device includes two mucoadhesive layers and two active agents, wherein the first active agent and the second active agent are both incorporated into the first mucoadhesive layer and the second active agent is also incorporated into the second mucoadhesive layer. It is to be understood that zero, one or more than one active agent may be included in each mucoadhesive layer of the devices disclosed herein, provided that at least one layer has at least one active agent.

In some embodiments, the device includes more than one mucoadhesive layer, one or more intermediate layers as described herein, and a single active agent incorporated into one or more mucoadhesive layers and/or intermediate layers. In some embodiments, the device includes more than one mucoadhesive layer, one or more intermediate layers as described herein, and more than one active agent incorporated into one or more mucoadhesive layers and/or one or more intermediate layers.

In some embodiments, the device includes more than one mucoadhesive layer as described herein such that the device adheres to the buccal mucosa and gum tissue, or any other mucosal surface of the oral cavity. In some embodiments, the device adheres to the sublingual mucosa. In certain embodiments the device, for example is adhered to the inner cheek and the gum, or the inner cheek, buccal mucosa and the retromolar trigone. In further embodiments, the device is administered, for example, beneath the tongue of a subject and adheres to the underside of the tongue and or frenulum and the floor of the oral cavity.

The device includes an abusable drug, which is buprenorphine, and an antagonist incorporated into any combination of layers discussed herein. In one embodiment, an antagonist and an abusable drug may be incorporated in the same mucoadhesive layer. In another embodiment, an antagonist is incorporated into a first mucoadhesive layer and an abusable drug incorporated into a second mucoadhesive layer. In yet a further embodiment, an antagonist is incorporated in two or more mucoadhesive layers and an abusable drug is incorporated into one of the mucoadhesive layers. In a further embodiment, an abusable drug is incorporated in two or more mucoadhesive layers and an antagonist is incorporated into one of the mucoadhesive layers. In one embodiment, an abusable drug is incorporated in one or more mucoadhesive layers and an antagonist is incorporated into one or more intermediate layers. In another embodiment, an antagonist is incorporated in one or more mucoadhesive layers and an abusable drug is incorporated into one or more intermediate layers. In a further embodiment, the abusable drug is incorporated into one or more mucoadhesive layers and one or more intermediate layers and the antagonist is incorporated into any one of the mucoadhesive or intermediate layers or incorporated into any combination of the mucoadhesive layers and the intermediate layers. In a further embodiment, the antagonist is incorporated into one or more mucoadhesive layers and one or more intermediate layers and the abusable drug is incorporated into any one of the mucoadhesive or intermediate layers or incorporated into any combination of the mucoadhesive layers and the intermediate layers. The antagonist in such embodiments can be associated with an abuse-resistant matrix as described in further detail herein. In some embodiments, the abuse-resistant matrix is an intermediate layer. In other embodiments the abuse-resistant matrix is an encapsulated form of the antagonist disbursed within any of the intermediate or outermost layers of the device. In certain embodiments, the abusable drug and the antagonist are incorporated such that they are inseparable, by pealing or any other mechanical means.

The devices and methods disclosed herein generally include an active agent. The term "active agent" refers to an agent to be incorporated into the devices and generally does not refer to the polymers employed to synthesize the mucoadhesive. Active agents include any compounds having a property of biological interest, e.g., ones that have a role in the life processes of a living organism. An active agent may be organic or inorganic, a monomer or a polymer, endogenous to a host organism or not, naturally occurring or synthesized in vitro and the like.

The present invention pertains to the active agent buprenorphine. According to one embodiment, a different active agent is additionally used. In this context, the active agent may comprise a single pharmaceutical or a combination of pharmaceuticals that are suitable for transmucosal and/or sublingual delivery. Pharmaceuticals include, but are not limited to abusable drugs, antagonists, antiinflammatory analgesic agents, steroidal anti- inflammatory agents, antihistamines, local anesthetics, bactericides, disinfectants, vasoconstrictors, hemostatics, chemotherapeutic drugs, antibiotics, keratolytics, cauterizing agents, and antiviral drugs antirheumatics, antihypertensives, bronchodilators, anticholinergics, antimenimic compounds, hormones, and macromolecules, peptides, proteins, vaccines, serotonin antagonists such as 5-HT3 antagonists, antianxiety agents, hypnotics, serotonin agonists such as 5-HT agonists or anti-migraine products. The amount of active agent to be used depends on the desired treatment strength and the composition of the layers, although preferably, the pharmaceutical component comprises from about 0.001 to about 99, more preferably from about 0.003 to about 30, and most preferably from about 0.005 to about 20% by weight of the device.

Examples of pharmaceuticals include, but are not limited to acetaminophen, methyl salicylate, monoglycol salicylate, aspirin, mefenamic acid, flufenamic acid, indomethacin, diclofenac, aiclofenac, diclofenac sodium, ibuprofen, ketoprofen, naproxen, pranoprofen, fenoprofen, sulindac, fenclofenac, clidanac, flurbiprofen, fentiazac, bufexamac, piroxicam, phenylbutazone, oxyphenbutazone, clofezone, pentazocine, mepirizole, tiaramide hydrochloride, hydrocortisone, predonisolone, dexamethasone, triamcinolone acetonide, fluocinolone acetonide, hydrocortisone acetate, predonisolone acetate, methyipredonisolone, dexamethasone acetate, betamethasone, betamethasone valerate, flumetasone, fluorometholone, beclomethasone diproprionate, diphenhydramine hydrochloride, diphenhydramine salicylate, diphenhydramine, chiorpheniramine hydrochloride, chlorphenirarnine maleate isothipendyl hydrochloride, tripelennamine hydrochloride, promethazine hydrochloride, methdilazine hydrochloride, dibucaine hydrochloride, dibucaine, lidocaine hydrochloride, lidocaine, benzocaine, p buthylaminobenzoic acid 2-(di-ethylamino) ethyl ester hydrochloride, procaine hydrochloride, tetracaine, tetracaine hydrochloride, chioroprocaine hydrochloride, oxyprocaine hydrochloride, mepivacaine, cocaine hydrochloride, piperocaine hydrochloride, dyclonine, dyclonine hydrochloride, thimerosal, phenol, thymol, benzalkonium chloride, benzethonium chloride, chlorhexidine, povidone iode, cetylpyridinium chloride, eugenol, trimethylammonium bromide, naphazoline nitrate, tetrahydrozoline hydrochloride, oxymetazoline hydrochloride, phenylephrine hydrochloride, tramazoline hydrochloride, thrombin, phytonadione, protamine sulfate, aminocaproic acid, tranexamic acid, carbazochrome, carbaxochrome sodium sulfanate, rutin, hesperidin, sulfamine, sulfathiazole, sulfadiazine, homosulfamine, sulfisoxazole, sulfisomidine, sulfamethizole, nitrofurazone, penicillin, meticillin, oxacillin, cefalotin, cefalordin, erythromcycin, lincomycin, tetracycline, chlortetracycline, oxytetracycline, metacycline, chloramphenicol, kanamycin, streptomycin, gentamicin, bacitracin, cycloserine, salicylic acid, podophyllum resin, podolifox, cantharidin, chloroacetic acids, silver nitrate, protease inhibitors, thymadine kinase inhibitors, sugar or glycoprotein synthesis inhibitors, structural protein synthesis inhibitors, attachment and adsorption inhibitors, and nucleoside analogues such as acyclovir, penciclovir, valacyclovir, and ganciclovir, ondansetron, granisetron and palonosetron, benzodiazepine derivatives, midazolam, clonazepam, alprazolam, zolpidem, eszopiclone, sumatriptan, zolmitriptan, naratriptan, frovatriptan, rizatriptan, almotriptan, eletriptan, and prochlorperazine.

In the present invention, the active agent is an abusable drug, which is buprenorphine.

In the present invention, the devices include a combination of an abusable drug and an antagonist. The abusable drug is buprenorphine and the antagonist is naloxone. In certain embodiments the devices contain an abusable drug, i.e. buprenorphine, and its antagonist such that abuse of buprenorphine is impeded. Thus, for example, illicit use efforts to extract buprenorphine from the transmucosal devices for parenteral injection (*e.g.*, by extraction of the drug by dissolving some or all of the transmucosal device in water or other solvent), are thwarted by the co-extraction of an antagonist. The antagonist is associated with an abuse-resistant matrix. The abuse-resistant matrix can be, but is not limited to a layer or coating, *e.g.,* a water-erodable coating or a water-hydrolysable matrix, *e.g.*, an ion exchange polymer, or any combination thereof. Thus, in one embodiment, the antagonist is associated with the matrix in a manner such that substantial amount is not released in the mouth. In another embodiment, the antagonist is adequately taste masked. The entrapment and/or taste masking may be achieved by physical entrapment by methods, such as microencapsulation, or by chemical binding methods, *e.g.*, by the use of a polymer that prevents or inhibits mucoabsorption of the antagonist, *e.g.,* ion exchange polymers. Without wishing to be bound by any particular theory, it is believed that the optimum formulation for the particular antagonist may be determined by understanding the ratios needed to prevent abuse, evaluating the possible binding mechanism, and evaluating the physico-chemical properties of the antagonists. In some embodiments, the antagonist is microencapsulated in an enteric polymer, polysaccharide, starch or polyacrylate. Microencapsulation can substantially prevent transmucosal absorption of the antagonist, and allow the subject to swallow the microencapsulated antagonist. The coating of the microcapsules can be designed to offer delayed release characteristics, but will release when the article or composition are placed in an aqueous environment, such as when the dosage form is chewed or subject to extraction. Delayed release can be accomplished, for example, by the use of starches or pH dependent hydrolysis polymers as coating materials for the microencapsulated antagonist. Starches, for example, would be susceptible to any enzymes that are present in the saliva, such as salivary amylase. In some embodiments, the antagonist is microencapsulated in a microcapsule or microsphere and then incorporated in the abuse resistant matrix. Such a microcapsule or microsphere containing antagonist may be comprised of polymers such as polyacrylates, polysaccharides, starch beads, polyactate beads, or liposomes. In a further embodiment, the microspheres and microcapsules are designed to release in specific parts of the small intestine. The amount of antagonist contained in the product can be chosen, for example, to block any psychopharmacological effects that would be expected from parenteral administration of the drug alone. In the present invention, the abusable drug is buprenorphine and the antagonist is naloxone. In some embodiments, the antagonist is associated with an abuse-resistant matrix as described herein. In some embodiments, the antagonist associated with an abuse-resistant matrix does not interfere with the transmucosal delivery of the abusable drug.

The antagonist incorporated into the abuse-resistant matrix includes, but is not limited to opiate or opioid antagonists, e.g., naloxone, naltrexone, nalmefene, nalide, nalmexone, nalorphine, naluphine, cyclazocine, levallorphan and physiologically acceptable salts and solvates thereof. In one embodiment, the abuse-resistant matrix comprises water soluble polymers, *e*.*g*., polymers similar to those described for the mucoadhesive and/or backing layers, but is associated with the device such that the antagonist is not mucosally absorbed to a significant extent. In some embodiments, the abuse-resistant matrix is a layer coating, *e.g.*, a water-erodable coating. That is, physical entrapment of the antagonist in the device, *e.g.*, the mucoadhesive layer, can be facilitated by a barrier layer which is coated with a water soluble polymer which erodes slowly. That is, antagonists may be at least partially coated or disposed within water-erodable coating. Methods of microencapsulation and particle coating have been defined in the literature. In some embodiments, the abuse-resistant matrix includes materials used for physical entrapment. Such materials include, but are not limited to, alginates, polyethylene oxide, poly ethylene glycols, polylactide, polyglycolide, lactide-glycolide copolymers, poly-epsilon-caprolactone, polyorthoesters, polyanhydrides and derivatives, methyl cellulose, ethyl cellulose, hydroxypropyl cellulose, hydroxyethyl cellulose, hydroxyethylmethyl cellulose, hydroxypropylmethyl cellulose, polyacrylic acid, and sodium carboxymethyl cellulose, poly vinyl acetate, poly vinyl alcohols, polyethylene glycol, polyethylene oxide, ethylene oxide-propylene oxide co-polymers, collagen and derivatives, gelatin, albumin, polyaminoacids and derivatives, polyphosphazenes, polysaccharides and derivatives, chitin, chitosan bioadhesive polymers, polyacrylic acid, polyvinyl pyrrolidone, sodium carboxymethyl cellulose and combinations thereof.

In some embodiments, the devices include an abusable drug and an antagonist that are less susceptible to abuse than an abusable drug alone. For example, when used in an abusive manner, the abusable drug may only retain about 50%, 40%, 30%, 20%, 10%, 5%, 2%, 1% or 0% of its efficacy, *e.g.*, as a pain reliever. Accordingly, when used in an abusive manner, it is believed that the effectiveness of the abusable drug, *e.g.*, the ability to produce a "high" in an addict, would be reduced by a corresponding amount, *e*.*g*., by about 50%, 60%, 70%, 80%, 90%, 95%, 98%, 99% or 100%.

The abuse-resistant matrix does not effectively release the antagonist when the device is used in a non-abusive manner. Without wishing to be bound by any particular theory, it is believed that the antagonist associated with an abuse-resistant matrix would not enter systemic circulation through the mucosa in a significant amount because it would be washed into the GI tract, *e*.*g*., swallowed. For example, the antagonist may be washed into the GI tract as either free-antagonist or as a coated or otherwise entrapped moiety, e.g., coated/entrapped by an ion-exchange polymer as described herein.

Conventional dosage forms including an opioid and an antagonist, *e.g.*, those described in U.S. Patent No. 4,582,384 and U.S. Patent No. 6,227,384, typically release the corresponding antagonist into the mucosa along with the opioid even when correctly administered. This impairs the activity of the opioid and it often becomes necessary to increase the quantity thereof required in the dosage form for satisfactory treatment of the patient. In these conventional dosage forms, the risk of undesirable accompanying symptoms is also increased in comparison to dosage forms which contain no opioid antagonists. Moreover, it is desirable not to further increase the stress on the patient by releasing a large proportion of opioid antagonist when such a dosage form is correctly administered.

In some embodiments, the abuse-resistant matrix is a layer or coating, *e.g.,* a water-erodable coating or layer at least partially disposed about the antagonist. In some embodiments, the abuse-resistant matrix is a water-hydrolysable, water-erodable or water-soluble matrix, *e*.*g*., an ion exchange polymer. The coating or water-hydrolysable matrix can be chosen such that it dissolves more slowly than a mucoadhesive layer as described above. The coating or water-hydrolysable matrix can additionally or alternatively be chosen such that it dissolves slowly enough not to release the antagonist at all.

The abuse-resistant matrix includes, but is not limited to, partially crosslinked polyacrylic acid, polycarbophil providone cross-linked sodium carboxymethylcellulose, gelatin, chitosan, AmberliteTM 1RP69, DuoliteTM AP143, AMBERLITE 1RP64, AMBERLITE 1RP88, and combinations thereof. In other embodiments, the abuse-resistant matrix includes, but is not limited to, alginates, polyethylene oxide, poly ethylene glycols, polylactide, polyglycolide, lactide-glycolide copolymers, poly-epsilon-caprolactone, polyorthoesters, polyanhydrides and derivatives, methyl cellulose, ethyl cellulose, hydroxypropyl cellulose, hydroxyethyl cellulose, hydroxyethylmethyl cellulose, hydroxypropylmethyl cellulose, polyacrylic acid, and sodium carboxymethyl cellulose, poly vinyl acetate, poly vinyl alcohols, polyethylene glycol, polyethylene oxide, ethylene oxide-propylene oxide co-polymers, collagen and derivatives, gelatin, albumin, polyaminoacids and derivatives, polyphosphazenes, polysaccharides and derivatives, chitin, or chitosan bioadhesive polymers, polyacrylic acid, polyvinyl pyrrolidone, sodium carboxymethyl cellulose, and combinations thereof. It is to be understood that polymers, layers, coatings, and water-hydrolyzable matrices are exemplary, and that additional abuse-resistant matrices can be envisioned using the teachings of the present disclosure.

In some embodiments, the abuse-resistant matrix is incorporated into one or more mucoadhesive layers and/or intermediate layers. In some embodiments, when the device is a multilayer disc or film, the abuse-resistant matrix is a layer or is incorporated into an intermediate layer which is disposed between mucoadhesive layers. In some embodiments, the abuse-resistant matrix is an intermediate layer. In some embodiments, the abuse-resistant matrix erodes at a slower rate than the one or more mucoadhesive layers and/or intermediate layers.

Where the device is abusively dissolved, for example, the antagonist and the abusable drug are released at substantially the same rate. For example, when the antagonist and the abusable drug are dissolved in water, they are both released at substantially the same rate. In other embodiments, the ratio of released antagonist to released drug is not less than about 1:20.

The antagonist incorporated into a device of any of the embodiments discussed herein is substantially transmucosally unavailable. Accordingly, the prescribed transmucosal administration of the drug containing device will not affect the availability of the active agent. The phrase "substantially transmucosally unavailable" refers to the fact that the antagonist in the compositions and devices is available transmucosally in amounts that do not effect, or negligibly effect, the efficacy of the abusable drug when employed in a non-abusive manner. Without wishing to be bound by any particular theory, it is believed that the antagonist is prevented or slowed from entering the system transmucosally while still being available for other routes of administration (*e*.*g*., swallowing or dissolution), thus allowing the abusable drug to act efficaciously in a transmucosal composition, but hindering the use of the composition in an abusive manner. That is, it is to be understood that the antagonist affects the efficacy of the abusable drug when the compositions disclosed herein are abused. In non-abusive situations, the antagonist provides no or negligible effect, *e.g.,* is swallowed. In some embodiments, less than about 25% antagonist (by weight versus abusable drug) can be delivered non-abusively, e.g., transmucosally. In other embodiments, less than about 15%, less than 5%, less than 2%, or less than about 1% antagonist is delivered transmucosally.

Methods are therefore provided for the use of a double-sided mucoadhesive device for the administration of buprenorphine wherein the device prohibits, discourages or prevents abuse of buprenorphine. The antagonist, as described above, acts to suppress the effects of buprenorphine when consumed by means other than by mucosal administration. In some embodiments, the extent of the absorption into systemic circulation of the antagonist by the subject is less than about 15% by weight. In some embodiments, the dosage of the drug is between about 50 and about 10 mg.

Plasticizers, flavoring and coloring agents, and preservatives may also be included in the outermost mucoadhesive layer or any of the intermediate layers. The amounts of each may vary depending on the drug or other components but typically these components comprise no more than 50%, preferably no more than 30%, most preferably no more than 15% by total weight of the device. In some embodiments, the device includes inactivating agents. In other embodiments, the device is substantially free of inactivating agents. As used herein, the term "inactivating agent" refers to a compound that inactivates or crosslinks the abusable drug, in order to decrease the abuse potential of the dosage form. Examples of inactivating agents include polymerizing agents, photoinitiators, and formalin. Examples of polymerizing agents include diisocyanates, peroxides, diimides, diols, triols, epoxides, cyanoacrylates, and UV activated monomers.

The devices can also optionally include a pharmaceutically acceptable dissolution-rate-modifying agent, a pharmaceutically acceptable disintegration aid (*e*.*g*., polyethylene glycol, dextran, polycarbophil, carboxymethyl cellulose, or poloxamers), a pharmaceutically acceptable plasticizer, a pharmaceutically acceptable coloring agent (*e.g.*, FD&C Blue #1), a pharmaceutically acceptable opacifier (*e*.*g*., titanium dioxide), pharmaceutically acceptable anti-oxidant (*e*.*g*., tocopherol acetate), a pharmaceutically acceptable system forming enhancer (*e.g.*, polyvinyl alcohol or polyvinyl pyrrolidone), a pharmaceutically acceptable preservative, flavorants (*e.g.*, saccharin and peppermint), neutralizing agents (*e.g.*, sodium hydroxide), buffering agents (*e*.*g*., monobasic, or tribasic sodium phosphate), or combinations thereof. Preferably, these components are individually present at no more than about 1% of the final weight of the device, but the amount may vary depending on the other components of the device.

The devices can also optionally include one or more plasticizers, to soften, increase the toughness, increase the flexibility, improve the molding properties, and/or otherwise modify the properties of the device. Plasticizers for use in the present embodiments can further include, e.g., those plasticizers having a relatively low volatility such as glycerin, propylene glycol, sorbitol, ethylene glycol, diethylene glycol, triethylene glycol, propylene glycol, polypropylene glycol, dipropylene glycol, butylene glycol, diglycerol, polyethylene glycol (*e.g.*, low molecular weight PEG), oleyl alcohol, cetyl alcohol, cetostearyl alcohol, and other pharmaceutical-grade alcohols and diols having boiling points above about 100°C at standard atmospheric pressure. Additional plasticizers include, *e.g.*, polysorbate 80, triethyl titrate, acetyl triethyl titrate, and tributyl titrate. Additional suitable plasticizers include, *e*.*g*., diethyl phthalate, butyl phthalyl butyl glycolate, glycerin triacetin, and tributyrin. Additional suitable plasticizers include, *e*.*g*., pharmaceutical agent grade hydrocarbons such as mineral oil (*e.g.*, light mineral oil) and petrolatum. Further suitable plasticizers include, *e.g.*, triglycerides such as medium-chain triglyceride, soybean oil, safflower oil, peanut oil, and other pharmaceutical agent grade triglycerides, PEGylated triglycerides such as Labrifil®, Labrasol® and PEG-4 beeswax, lanolin, polyethylene oxide (PEO) and other polyethylene glycols, hydrophobic esters such as ethyl oleate, isopropyl myristate, isopropyl palmitate, cetyl ester wax, glyceryl monolaurate, and glyceryl monostearate.

One or more disintegration aids can optionally be employed to increase the disintegration rate and shorten the residence time of the subject devices. Disintegration aids useful herein include, *e*.*g*., hydrophilic compounds such as water, methanol, ethanol, or low alkyl alcohols such as isopropyl alcohol, acetone, methyl ethyl acetone, alone or in combination. Specific disintegration aids include those having less volatility such as glycerin, propylene glycol, and polyethylene glycol.

One or more dissolution-rate-modifying agents can optionally be employed to decrease the disintegration rate and lengthen the residence time of the device provided herein. Useful dissolution-rate modifying agents include, *e.g.*, hydrophobic compounds such as heptane, and dichloroethane, polyalkyl esters of di- and tricarboxylic acids such as succinic and citric acid esterified with C₆ to C₂₀ alcohols, aromatic esters such as benzyl benzoate, triacetin, propylene carbonate and other hydrophobic compounds that have similar properties. These compounds can be used alone or in combination in the device. The residence time of the subject device depends on the erosion rate of the water erodable polymers used in the formulation and their respective concentrations. The erosion rate may be adjusted, for example, by mixing together components with different solubility characteristics or chemically different polymers, such as hydroxyethyl cellulose and hydroxypropyl cellulose; by using different molecular weight grades of the same polymer, such as mixing low and medium molecular weight hydroxyethyl cellulose; by using excipients or plasticizers of various lipophilic values or water solubility characteristics (including essentially insoluble components); by using water soluble organic and inorganic salts; by using crosslinking agents such as glyoxal with polymers such as hydroxyethyl cellulose for partial crosslinking; or by post-treatment irradiation or curing, which may alter the physical state of the film, including its crystallinity or phase transition, once obtained. These strategies might be employed alone or in combination in order to modify the erosion kinetics of the device.

In some embodiments, the device is bioerodable. The use of erodable components allows the device to erode over a period of time, with natural bodily fluids dissolving or eroding away the carrier, while the pharmaceutical remains at the application site. Unlike bandages and other non-water-erodable film systems, the user of the instant devices does not have to remove the device following treatment. The user also does not experience any substantial sensation of the presence of a foreign object at the mucosal surface or within the body cavity, given that upon application, water absorption softens the device, and over time, the device slowly dissolves or erodes away. In some embodiments, the device erodes in the oral cavity. In some embodiments, the device does not leave any substantial residue and therefore contributes to a significant decrease in nausea a subject might experience from the administration of other devices. The term "bioerodable" as used herein refers to a property of a device that allows a solid or semisolid portion of a device to sufficiently degrade by surface erosion, bioerosion, and/or bulk degradation such that it is small enough to be swallowed without causing system irritations such as nausea. Bulk degradation is the process in which a material, *e.g.,* a polymer, degrades in a fairly uniform manner throughout the matrix. This results in a reduction of molecular weight (Ms) without immediate change in physical properties, followed by fragmentation due to faster penetration of saliva or water into the device than conversion of the device into saliva- or water-soluble form. Bioerosion or surface erosion generally occurs when the rate at which saliva or water penetrates the material is slower than the rate of the conversion of the material into saliva- or water-soluble substances. Bioerosion generally results in a thinning of the material over time, though the bulk integrity is maintained. It is to be understood that "bioerodable" refers to the device as a whole, and not necessarily to its individual components. For example, if an antagonist is microencapsulated or coated and then incorporated into one of the instant devices, the microcapsules or coating may or may not be bioerodable, but the device as a whole may be bioerodable such that as the device is eroded the intact microcapsules or coated antagonist is swallowed. This can be advantageous because the device may erode and the microcapsules or coated antagonist can be delivered to the GI tract intact, *i*.*e*., without crossing the mucosa. The term "bioerodable" is intended to encompass many modes of material removal, such as enzymatic and non-enzymatic hydrolysis, oxidation, enzymatically-assisted oxidation, wear, degradation and/or dissolution. Without wishing to be bound by any particular theory, it is believed that bioerodable devices may be advantageous because such devices do not have to be removed after use.

Bioerodable materials are generally selected on the basis of their degradation characteristics to provide a sufficient residence time or functional lifespan for the particular application. In some devices, a functional lifespan of between about 1 minute and about 10 hours may be suitable. In some embodiments, the functional lifespan is about 20 minutes. In some embodiments, the functional lifespan is about 5 minutes, about 10 minutes, about 15 minutes, about 20 minutes, about 30 minutes, about 45 minutes, about 60 minutes, about 2 hours, about 3 hours, about 4 hours, about 5 hours, or about 10 hours. All ranges and values which fall between the ranges and values listed herein are meant to be encompassed by the present device embodiments disclosed herein. For example, residence times of between about 5 minutes and about 45 minutes, between about 6 minutes and about 53 minutes, between about 13 minutes and about 26 minutes, etc. are all encompassed herein. Shorter or longer periods may also be appropriate.

Bioerodable materials include, but are not limited to, polymers, copolymers and blends of polyanhydrides (*e*.*g*., those made using melt condensation, solution polymerization, or with the use of coupling agents, aromatic acids, aliphatic diacids, amino acids, *e.g.,* asp artic acid and glutamic acid, and copolymers thereof); copolymers of epoxy terminated polymers with acid anhydrides; polyorthoesters; homo- and copolymers of a-hydroxy acids including lactic acid, glycolic acid, -caprolactone, y butyrolactone, and -valerolactone; homo- and copolymers of a-hydroxy alkanoates; polyphosphazenes; polyoxyalkylenes, *e.g.*, where ailcene is 1 to 4 carbons, as homopolymers and copolymers including graft copolymers; poly(amino acids), including pseudo poly(amino acids); polydioxanones; and copolymers of polyethylene glycol with any of the above.

The present invention also provides for a device that is formed in part from a thin and flexible mucoadhesive film. The device and film are bioerodable. Preparation of the film, in certain aspects, employs water-soluble polymers which include, but are not limited to, hydroxyethyl cellulose, hydroxypropyl cellulose, hydroxypropylmethyl cellulose, hydroxyethylmethyl cellulose, polyacrylic acid (PAA) which may or may not be partially crosslinked, sodium carboxymethyl cellulose (NaCMC), and polyvinylpyrrolidone (PVP), or combinations thereof. Other mucoadhesive bioerodable polymers may also be used in the present invention. The mucoadhesive layer may comprise at least one film-forming water-erodable polymer (the "film-forming polymer") and at least one pharmacologically acceptable polymer known for its bioadhesive capabilities (the "bioadhesive polymer"). The film forming polymer may comprise hydroxyethyl cellulose, hydroxypropyl cellulose, hydroxypropylmethyl cellulose, hydroxyethyl methyl cellulose, polyvinyl alcohol, polyethylene glycol, polyethylene oxide, ethylene oxide-propylene oxide co-polymers, collagen and derivatives, gelatin, albumin, polyaminoacids and derivatives, polyphosphazenes, polysaccharides and derivatives, chitin and chitosan, alone or in combination. Preferably, the film-forming polymer comprises hydroxyethyl cellulose and hydroxypropyl cellulose. Preferably, in the case of hydroxyethyl cellulose, the average molecular weight (Mw estimated from intrinsic viscosity measurements) is in the range 10² to 10⁶ and more preferably in the range 10³ to 10⁵, while in the case of hydroxypropyl cellulose, the average molecular weight (Mw obtained from size exclusion chromatography measurements) is in the range 50×10³ to 1.5×10⁶, and more preferably between 80×10³ to 5×10⁵. The ratio of the bioadhesive polymer to the film-forming polymer in the adhesive layer may vary, depending on the type of pharmaceutical and the amount of pharmaceutical to be used.

In some embodiments, the intermediate layers are generally comprised of bioerodable, film- forming pharmaceutically acceptable polymers which include, but are not limited to, hydroxyethyl cellulose, hydroxypropyl cellulose, hydroxypropylmethyl cellulose, hydroxyethylmethyl cellulose, polyvinylalcohol, polyethylene glycol, polyethylene oxide, ethylene oxide-propylene oxide co-polymers, or combinations thereof. The intermediate layer may comprise other water-soluble, film-forming polymers as known in the art. Exemplary layers, including polymers suitable for such layers are also described, *e*.*g*., in U.S. Patent Nos. 5,800,832 and 6,159,498, the entireties of which are incorporated herein by this reference.

The devices of the present invention can include various forms. For example, the device can be a disc or film. In one embodiment, the device comprises a mucoadhesive disc. In one embodiment of the methods and devices of the present invention, the device is a flexible device. The thickness of the device of the present invention, in its form as a solid film or disc, may vary depending on the thickness of each of the layers. Typically, the thickness ranges from about 0.005 mm to about 3 mm, and more specifically, from about 0.05 mm to about 0.5 mm. When the device is multi layered, the thickness of each layer can vary from about 10% to about 90% of the overall thickness of the device, and specifically can vary from about 30% to about 60% of the overall thickness of the device.

In some embodiments, there is relatively minimal mouth feel and little discomfort because of the thinness and flexibility of the devices as compared to conventional tablet or lozenge devices. This is especially advantageous for patients who have inflammation of the mucosa and/or who may otherwise not be able to comfortably use conventional devices. The devices of the present invention are small and flexible enough so that they can adhere to a non-inflamed area of the mucosa and still be effective, *i.e.*, the mucosa does not need to be swabbed with the device of the present invention.

In various embodiments, the devices of the present invention can be in any form or shape such as a sheet or disc, circular or square in profile or cross-section, *etc.*, provided the form allows for the delivery of the active agent to the subject. In some embodiments, the devices of the present invention can be scored, perforated or otherwise marked to delineate certain dosages.

The devices of the present invention can be adapted for any method of mucosal administration. In some embodiments of the invention, methods are provided wherein the device is adapted for buccal administration and/or sublingual administration. In certain methods, the devices provided herein are administered to a subject in need thereof by adhering the device to a mucosal surface. By way of example, the device may be used in methods of treating pain, wherein the device is affixed or adhered to any mucosal surface of a subject wherein the device's two mucoadhesive surfaces may be affixed to two or more mucosal surfaces. The incorporated buprenorphine is delivered transmucosally by diffusion from the device, across the mucoadhesive surface, to the mucosal surface across the mucosal surface and is systemically circulated. Also provided are methods of locally protecting irritated or damaged mucosal tissue by applying the double-sided mucoadhesive devices of the present invention to the irritated or damaged tissue. Buprenorphine may be locally administered and/or systemically administered to the protected site.

In other aspects, methods for the transmucosal delivery of buprenorphine to two or more mucosal surfaces are provided using any of the devices discussed herein. Buprenorphine can diffuse from the device to two or more sites of adhesion either simultaneously, substantially simultaneously or sequentially. In certain further embodiments, the diffusion to the points of adhesion is simultaneous. In some embodiments, the method includes applying a device of the invention as described herein to a mucosal cavity of a subject such that there is adhesion of the delivery device to at least two surfaces of the mucosal cavity and diffusion of an active agent across at least two surfaces of the mucosal cavity.

In some embodiments, the present invention is directed to devices for use in methods for treating pain in a subject, *e.g.*, a human, with a dosage of buprenorphine. The methods can employ any of the devices enumerated herein with any of the desired release profiles herein. In some embodiments, buprenorphine is delivered to the subject in less than about 1 hour, 30 minutes, 20 minutes or 10 minutes. In some embodiments, buprenorphine is systemically delivered. While not wishing to be bound by a single theory, it is believed that the double-sided mucoadhesive devices of the invention described herein facilitates improved bioavailability of buprenorphine to the subject due to the multiple points of adhesion that are available with the multiple mucoadhesive surfaces. It may also be found that the typically expected nausea side-effects are diminished. The present invention also provides devices wherein the amount of buprenorphine that can be loaded into the device is substantially greater than with conventional devices. The buprenorphine load ranges from 0.01 mg to about 60 mg and more preferably between 0.1 mg and 30 mg.

Due to the addictive nature of certain opioids used in the treatment of pain, including buprenorphine, it is found that addiction can be moderated by the use of the devices described herein. Accordingly, devices for use in methods for the treatment of addiction to opioids are provided herein, by administering buprenorphine to the subject using any of the devices provided herein. For example, embodiments are provided wherein the double-sided mucoadhesive device includes an abuse-resistant matrix. The abuse resistant matrix prevents, inhibits or discourages abusive use. The abuse-resistant matrix is formulated such that transmucosal administration provides the prescribed amount of buprenorphine contained therein. However, where an addicted subject would seek to administer more than the prescribed dosage by means such as dissolving the device and injecting the dissolved buprenorphine, the abuse resistant matrix would render the buprenorphine substantially ineffective. Accordingly, potential abuse would be discouraged while prescribed use would be encouraged.

The dual points of adhesion also allows the device to deliver buprenorphine to the mucosa so as to achieve onset of pain relief in less than about 0.5, 0.3, 0.2, or 0.1 hours. The dual points of adhesion available on a double-sided mucoadhesive device as described herein, allows for the delivery of buprenorphine directly to the mucosa to achieve a Tₘₐₓ of less than about 1.75 hours, less than about 1.5 hours, less than about 1.0 hour, less than about 0.5 hours or less than 0.25 hours. In some embodiments, the AUC₀₋₂₄ is about 10 hr-ng/mL or about 12.5 hr-ng/mL or about 15 hr-ng/mL. In some embodiments, adherence of the devices of the present invention to the mucosal surface occurs in about five seconds. In some embodiments, the devices of the present invention naturally erode in about twenty to thirty minutes, without any need to hold the device in place. Exemplary embodiments of the present invention encompasses devices wherein buprenorphine is in the amounts of about 0.1 mg to about 60 mg such that Tₘₐₓ is less than about 100 minutes, less than about 80 minutes, less than about 60 minutes, less than about 30 minutes or less than about 20 minutes. Where the device contains about 16 mg of buprenorphine, Cₘₐₓ may be about 8.0 ng/mL. Where the device contains 8 mg of buprenorphine, Cₘₐₓ may be about 4.5 ng/mL. Where the device contains about 4 mg of buprenorphine, Cₘₐₓ may be about 2.5 ng/mL.

The pharmaceutical delivery devices of the present invention may be prepared by various methods known in the art. For example, in one embodiment, the components are dissolved in the appropriate solvent or combination of solvents to prepare a solution. Solvents for use in the present invention may comprise water, methanol, ethanol, or lower alkyl alcohols such as isopropyl alcohol, acetone, ethyl acetate, tetrahydrofuran, dimethyl sulfoxide, or dichloromethane, or any combination thereof. The residual solvent content in the dried, multilayered film may act as a plasticizer, an erosion- or dissolution -rate- modifying agent or may provide some pharmaceutical benefit. Desired residual solvent may reside in the layers.

A solution is then coated onto a substrate, *e.g.*, cast and processed into a thin film by techniques known in the art, such as film coating, film casting, spin coating, or spraying using the appropriate substrate. The thin film is then dried. The drying step can be accomplished in any type of oven; however, the amount of residual solvent depends on the drying procedure. Where multiple layers are desired, such layers may be filmed independently and then laminated together or may be filmed one on the top of the other. The film obtained after the layers have been laminated together or coated on top of each other may be cut into any type of shape, for application to the mucosal tissue. Some shapes include disks, ellipses, squares, rectangles, and parallepipedes.

### EXEMPLIFICATION

### Prospective Example 1: Preparation of Exemplary Mucoadhesive Device (not according to the present invention)

An exemplary mucoadhesive device of the present invention will be prepared by adding water (about 89% total formulation, by weight) to a mixing vessel followed by sequential addition of propylene glycol (about 0.5% total formulation, by weight), sodium benzoate (about 0.06% total formulation, by weight), methylparaben (about 0.1% total formulation, by weight) and propylparaben (about 0.03% total formulation, by weight), vitamin E acetate (about 0.01% total formulation, by weight) and citric acid (about 0.06% total formulation, by weight), red iron oxide (about 0.01% total formulation, by weight), and monobasic sodium phosphate (about 0.04% total formulation, by weight). After the components are dispersed and/or dissolved, 800 µg fentanyl citrate (about 0.9% total formulation, by weight) will be added, and the vessel will be heated to about 120 to 130°F. After dissolution, the polymer mixture [hydroxypropyl cellulose (Klucel EF, about 0.6% total formulation, by weight), hydroxyethyl cellulose (Natrosol 250L, about 1.9% total formulation, by weight), polycarbophil (Noveon AA1 (about 0.6% total formulation, by weight), and carboxy methyl cellulose (Aqualon 7LF, about 5.124% total formulation, by weight)] will be added to the vessel, and stirred until dispersed. Subsequently, heat will be removed from the mixing vessel. Tribasic sodium phosphate and sodium hydroxide may then be added to adjust the blend to a desired pH. The blend will be mixed under vacuum for a few hours and stored in an air-sealed vessel until its use in the coating operation.

One or more layers will be cast in series onto a suitable surface, *e*.*g*., St. Gobain polyester liner. The blend as prepared above will be cast onto the liner, cured in an oven at about 65°C to 95°C, and dried. Additional layers can be cast on top of the initial layer using the same procedure. The devices will then be die-cut, *e*.*g*., by kiss-cut method and removed from the casting surface. The devices can be configured, for example, in the form of a disc, rectangular in shape with round corners. Multiple layers may be bonded together such as to avoid delamination during or after application to mucosal surfaces.

### Prospective Example 2: Preparation of Mucoadhesive Layer

The mucoadhesive layer will be prepared by adding water to a mixing vessel followed by sequential addition of propylene glycol, sodium benzoate, methylparaben and propylparaben, vitamin E acetate, citric acid, yellow iron oxide, and monobasic sodium phosphate. After the components are dispersed and/or dissolved, fentanyl or buprenorphine is added, and the vessel is heated to 120 to 130°F. The polymer mixture - hydroxypropyl cellulose (Klucel EF), hydroxyethyl cellulose (Natrosol 250L), polycarbophil (Noveon AA1), and carboxy methyl cellulose (Aqualon 7LF) - will then be added to the vessel, and stirred until dispersed. Subsequently, heat will be removed from the mixing vessel. As the last addition step, tribasic sodium phosphate (buffering agent) and sodium hydroxide (pH adjusting agent) will be added to adjust the blend to a desired pH. The blend will then be mixed under vacuum for a few hours. The prepared mixture will be stored in an air-sealed vessel until it is ready for use in the coating operation. The % w/w for each ingredient, and any additional ingredients, are illustrated in the following tables (1, 2 and 3). Table 1, represents formulation 1 of the device with buprenorphine, while Table 2 provides the details of formulation 2 containing buprenorphine. Table 3 contains a third formulation containing fentanyl citrate. For formulation 1, the pH ranges from 4.5 to 5.5. For formulation 2, the pH ranges from 5.0 to 6.0.

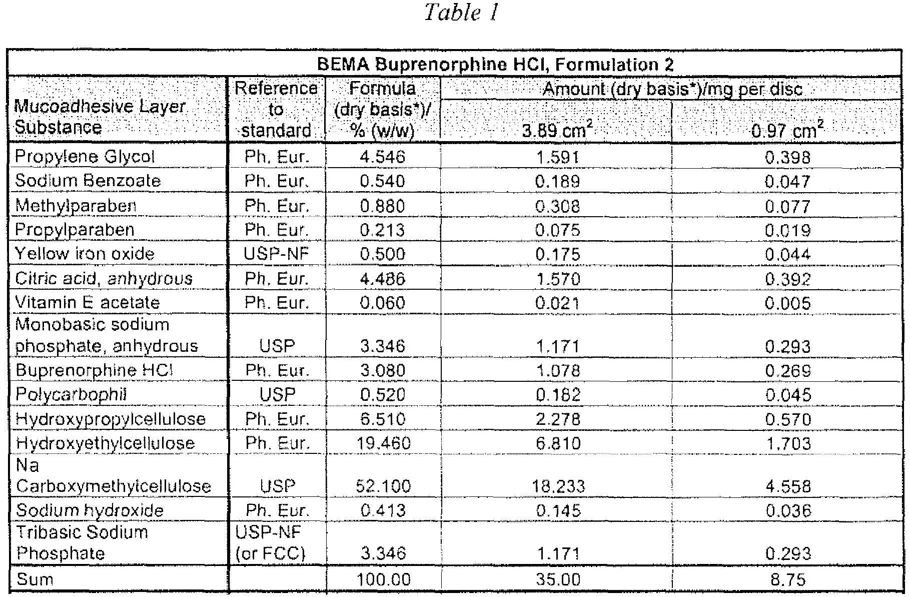

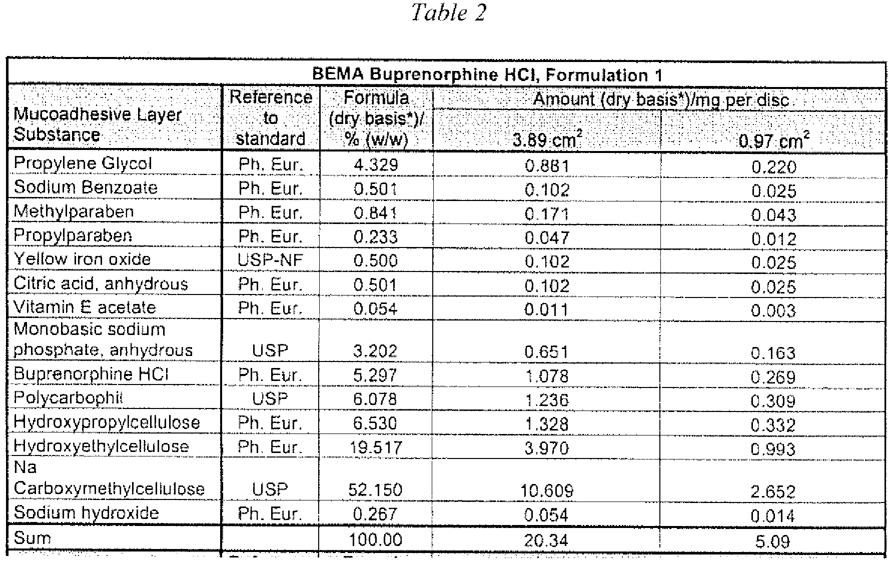

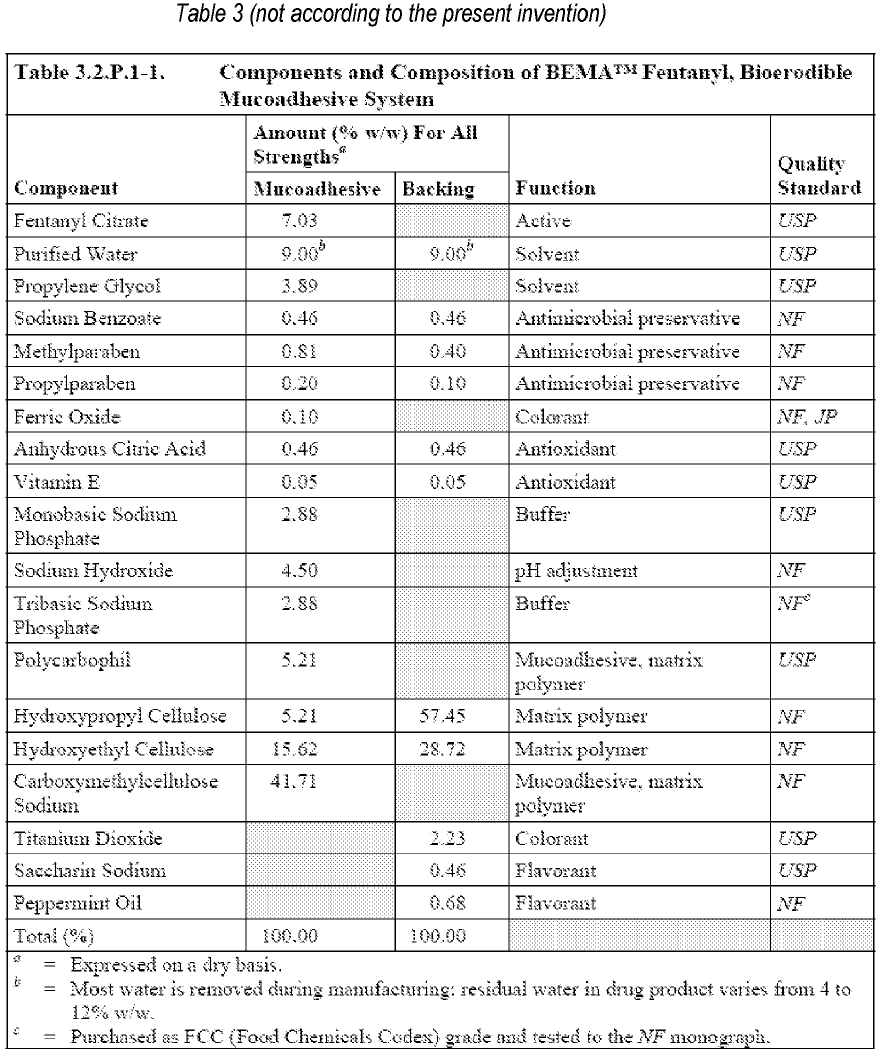

The coating process will involve casting the layer(s) in series onto a St. Gobain polyester liner. A first mucoadhesive layer will be cast using a knife-on-a-blade coating method. Subsequently, if desired, a second mucoadhesive layer will be cast onto the first mucoadhesive layer, cured in an oven at about 65 to 95 °C and dried. The product can then be die-cut by kiss-cut method and removed from the casting surface.

## Claims

1. A thin and flexible pharmaceutical delivery device for use in a method of transmucosally delivering buprenorphine to a subject, wherein the pharmaceutical delivery device comprises:
a first mucoadhesive surface for transmucosal delivery of buprenorphine;
a second mucoadhesive surface, opposing the first mucoadhesive surface, for transmucosal delivery of buprenorphine; and
an abusable drug and an antagonist incorporated into the device,
wherein the abusable drug is buprenorphine and the antagonist is naloxone, and
wherein the first and second mucoadhesive surfaces are defined by at least one thin and flexible mucoadhesive film layer; and
wherein an effective amount of buprenorphine is delivered to the subject.

2. The device of claim 1, wherein the device is bioerodable.

3. The device of claim 1, wherein the first and second mucoadhesive surfaces are defined by a single mucoadhesive layer.

4. The device of claim 1, wherein the first and second mucoadhesive surfaces are defined by at least a first and a second thin and flexible mucoadhesive film layer, and wherein the device preferably further comprises an intermediate layer disposed between the two mucoadhesive surfaces, said intermediate layer preferably fulfilling a feature selected from the following:
(i) the intermediate layer comprises an abuse-resistant matrix and an antagonist associated with the abuse-resistant matrix such that the antagonist is substantially transmucosally unavailable;
(ii) the intermediate layer comprises a microencapsulated antagonist; and
(iii) the intermediate layer is occlusive to buprenorphine.

5. The device of claim 1, wherein the mucoadhesive layer comprises microencapsulated antagonist.

6. The device of claim 4, wherein buprenorphine is incorporated into the first mucoadhesive layer, and the second mucoadhesive layer.

7. The device of claim 1, wherein the device is adapted for transmucosal delivery of buprenorphine to two or more mucosal surfaces,
wherein the device is preferably administered to a subject by applying the device to a mucosal cavity of the subject such that there is adhesion of the delivery device to at least two surfaces of the mucosal cavity and diffusion of the active agent across the first mucoadhesive surface and the second mucoadhesive surface, and
wherein the effective amount of buprenorphine is more preferably delivered to the subject in less than about 1 hour, and even more preferably in less than about 30 minutes.

8. The device of claim 1, wherein the device contains between about 0.1 mg to about 60 mg of buprenorphine, and
wherein (a) Tₘₐₓ is preferably less than about 100 minutes, more preferably less than about 80 minutes, even more preferably less than about 60 minutes, yet more preferably less than about 30 minutes and most preferably less than about 20 minutes, or
wherein (b) preferably more than 30% of the buprenorphine, more preferably more than 50% of the buprenorphine and most preferably more than 60% of the buprenorphine becomes bioavailable.

9. The device of claim 2, wherein the device has a functional lifespan between about 6 minutes and about 53 minutes, preferably, between about 13 minutes and about 26 minutes.

10. The device according to any of the preceding claims, wherein the device is adapted for treatment of addiction or pain.

11. The device of claim 10, wherein the pain is breakthrough cancer pain.

12. The device according to any of the preceding claims wherein the effective amount of buprenorphine is delivered to the subject in one unit dose.

## Patentansprüche

1. Dünne und flexible pharmazeutische Abgabevorrichtung zur Verwendung in einem Verfahren zur transmukosalen Abgabe von Buprenorphin an ein Individuum, wobei die pharmazeutische Abgabevorrichtung umfasst:
eine erste mukoadhäsive Oberfläche zur transmukosalen Abgabe von Buprenorphin;
eine zweite mukoadhäsive Oberfläche, die der ersten mukoadhäsiven Oberfläche gegenüberliegt, zur transmukosalen Abgabe von Buprenorphin; und
ein missbräuchlich verwendbares Arzneimittel und ein Antagonist, die in die Vorrichtung integriert sind,
wobei das missbräuchlich verwendbare Arzneimittel Buprenorphin ist und der Antagonist Naloxon ist, und
wobei die erste und die zweite mukoadhäsive Oberfläche durch mindestens eine dünne und flexible mukoadhäsive Filmschicht definiert sind; und
wobei eine wirksame Menge Buprenorphin an das Individuum abgegeben wird.

2. Vorrichtung gemäß Anspruch 1, wobei die Vorrichtung bioerodierbar ist.

3. Vorrichtung gemäß Anspruch 1, wobei die erste und die zweite mukoadhäsive Oberfläche durch eine einzelne mukoadhäsive Schicht definiert sind.

4. Vorrichtung gemäß Anspruch 1, wobei die erste und die zweite mukoadhäsive Oberfläche durch mindestens eine erste und eine zweite dünne und flexible mukoadhäsive Filmschicht definiert sind, und wobei die Vorrichtung vorzugsweise ferner eine intermediäre Schicht umfasst, die zwischen den beiden mukoadhäsiven Oberflächen angeordnet ist, wobei die intermediäre Schicht vorzugsweise ein Merkmal erfüllt, das aus den folgenden ausgewählt ist:
(i) die intermediäre Schicht umfasst eine missbrauchsresistente Matrix und einen Antagonisten, der mit der missbrauchsresistenten Matrix so assoziiert ist, dass der Antagonist im Wesentlichen transmukosal nicht verfügbar ist;
(ii) die intermediäre Schicht umfasst einen mikroverkapselten Antagonisten; und
(iii) die intermediäre Schicht ist für Buprenorphin okklusiv.

5. Vorrichtung gemäß Anspruch 1, wobei die mukoadhäsive Schicht einen mikroverkapselten Antagonisten umfasst.

6. Vorrichtung gemäß Anspruch 4, wobei Buprenorphin in die erste mukoadhäsive Schicht und in die zweite mukoadhäsive Schicht integriert ist.

7. Vorrichtung gemäß Anspruch 1, wobei die Vorrichtung zur transmukosalen Abgabe von Buprenorphin an zwei oder mehr Schleimhautoberflächen vorgesehen ist,
wobei die Vorrichtung vorzugsweise an ein Individuum verabreicht wird, indem die Vorrichtung in einem Schleimhauthohlraum des Individuums aufgetragen wird, so dass die Abgabevorrichtung an mindestens zwei Oberflächen des Schleimhauthohlraums haftet und der Wirkstoff über die erste mukoadhäsive Oberfläche und die zweite mukoadhäsive Oberfläche diffundiert, und
wobei die wirksame Menge an Buprenorphin besonders bevorzugt in weniger als 1 Stunde und stärker bevorzugt in weniger als ungefähr 30 Minuten an das Individuum abgegeben wird.

8. Vorrichtung gemäß Anspruch 1, wobei die Vorrichtung zwischen ungefähr 0,1 mg bis ungefähr 60 mg Buprenorphin enthält, und
wobei (a) Tₘₐₓ vorzugsweise weniger als ungefähr 100 Minuten, mehr bevorzugt weniger als ungefähr 80 Minuten, stärker bevorzugt weniger als ungefähr 60 Minuten, noch stärker bevorzugt weniger als ungefähr 30 Minuten und am stärksten bevorzugt weniger als 20 Minuten beträgt, oder
wobei (b) vorzugsweise mehr als 30% des Buprenorphins, stärker bevorzugt mehr als 50% des Buprenorphins und am stärksten bevorzugt mehr als 60% des Buprenorphins bioverfügbar wird.

9. Vorrichtung gemäß Anspruch 2, wobei die Vorrichtung eine funktionelle Lebenszeit von zwischen ungefähr 6 Minuten und ungefähr 53 Minuten, vorzugsweise zwischen ungefähr 13 Minuten und ungefähr 26 Minuten aufweist.

10. Vorrichtung gemäß einem der vorherigen Ansprüche, wobei die Vorrichtung zur Behandlung einer Abhängigkeit oder von Schmerzen vorgesehen ist.

11. Vorrichtung gemäß Anspruch 10, wobei der Schmerz krebsbedingter Durchbruchsschmerz ist.

12. Vorrichtung gemäß einem der vorherigen Ansprüche, wobei die wirksame Menge an Buprenorphin in einer Einheitsdosis an das Individuum abgegeben wird.

## Revendications

1. Dispositif d'administration pharmaceutique mince et flexible pour une utilisation dans une méthode d'administration par voie transmucosale de buprénorphine à un sujet, dans lequel le dispositif d'administration pharmaceutique comprend :
une première surface muco-adhésive pour l'administration transmucosale de buprénorphine ;
une deuxième surface muco-adhésive, opposée à la première surface muco-adhésive, pour l'administration transmucosale de buprénorphine ; et
un médicament pouvant faire l'objet d'abus et un antagoniste incorporés dans le dispositif,
dans lequel le médicament pouvant faire l'objet d'abus est la buprénorphine et l'antagoniste est la naloxone, et
dans lequel les première et deuxième surfaces muco-adhésives sont définies par au moins une couche de film muco-adhésif mince et flexible ; et
dans lequel une quantité efficace de buprénorphine est administrée au sujet.

2. Dispositif selon la revendication 1, dans lequel le dispositif est bio-érodable.

3. Dispositif selon la revendication 1, dans lequel les première et deuxième surfaces muco-adhésives sont définies par une couche muco-adhésive unique.

4. Dispositif selon la revendication 1, dans lequel les première et deuxième couches muco-adhésives sont définies par au moins une première et une deuxième couche de film muco-adhésif mince et flexible, et dans lequel le dispositif comprend en outre de préférence une couche intermédiaire disposée entre les deux surfaces muco-adhésives, ladite couche intermédiaire satisfaisant de préférence une caractéristique sélectionnée parmi les suivantes :
(i) la couche intermédiaire comprend une matrice résistante aux abus et un antagoniste associé à la matrice résistante aux abus de sorte que l'antagoniste soit essentiellement indisponible par voie transmucosale ;
(ii) la couche intermédiaire comprend un antagoniste microencapsulé ; et
(iii) la couche intermédiaire est occlusive à la buprénorphine.

5. Dispositif selon la revendication 1, dans lequel la couche muco-adhésive comprend l'antagoniste microencapsulé.

6. Dispositif selon la revendication 4, dans lequel la buprénorphine est incorporée dans la première couche muco-adhésive, et la deuxième couche muco-adhésive.

7. Dispositif selon la revendication 1, dans lequel le dispositif est adapté pour une administration transmucosale de buprénorphine à deux surfaces mucosales ou plus,
dans lequel le dispositif est administré de préférence à un sujet en appliquant le dispositif sur une cavité mucosale du sujet de sorte qu'il y ait une adhérence du dispositif d'administration à au moins deux surfaces de la cavité mucosale et la diffusion de l'agent actif à travers la première surface muco-adhésive et la deuxième surface muco-adhésive, et
dans lequel la quantité efficace de buprénorphine est de manière plus préférée administrée au sujet en moins d'environ 1 heure, et de manière encore plus préférée en moins d'environ 30 minutes.

8. Dispositif selon la revendication 1, dans lequel le dispositif contient entre environ 0,1 mg à environ 60 mg de buprénorphine, et
dans lequel (a) Tₘₐₓ est de préférence moins d'environ 100 minutes, de manière plus préférée moins d'environ 80 minutes, de manière encore plus préférée moins d'environ 60 minutes, de manière toujours plus préférée moins d'environ 30 minutes et de la manière la plus préférée moins d'environ 20 minutes, ou dans lequel (b) de préférence plus de 30 % de la buprénorphine, de manière plus préférée plus de 50 % de buprénorphine et de la manière la plus préférée plus de 60 % de la buprénorphine devient biodisponible.

9. Dispositif selon la revendication 2, dans lequel le dispositif a une durée de vie fonctionnelle entre environ 6 minutes et environ 53 minutes, de préférence, entre environ 13 minutes et environ 26 minutes.

10. Dispositif selon l'une quelconque des revendications précédentes, dans lequel le dispositif est adapté pour le traitement de l'addiction ou de la douleur.

11. Dispositif selon la revendication 10, dans lequel la douleur est une douleur cancéreuse avancée.

12. Dispositif selon l'une quelconque des revendications précédentes dans lequel la quantité efficace de buprénorphine est administrée au sujet en une dose unitaire.
